**Europäisches Patentamt**

**European Patent Office**

**Office européen des brevets**

(19)

(11) Numéro de publication: **0 016 890**
**A1**

(12) # DEMANDE DE BREVET EUROPEEN

(21) Numéro de dépôt: **79400195.8**

(22) Date de dépôt: **27.03.79**

(51) Int. Cl.³: **A 61 K 9/52,** A 61 K 31/44

---

(43) Date de publication de la demande: **15.10.80**
**Bulletin 80/21**

(71) Demandeur: **Cherqui, Jean Sadia, 55, Rue Pergolèse,**
**F-75016 Paris (FR)**
Demandeur: **Djiane, Alain Claude, 105, Avenue du Roule,**
**F-92200 Neuilly S/Seine (FR)**

(72) Inventeur: **Cherqui, Jean Sadia, 55, Rue Pergolèse,**
**F-75016 Paris (FR)**
Inventeur: **Djiane, Alain Claude, 105, Avenue du Roule,**
**F-92200 Neuilly S/Seine (FR)**

(74) Mandataire: **Burtin, Jean-François, 5 Bis, rue**
**Parmentier, F-92200 Neuilly S/Seine (FR)**

(84) Etats contractants désignés: **BE CH DE GB IT LU NL SE**

---

(54) **Nouvelle forme galénique d'administration de la bêtahistine.**

(57) Nouvelle forme galénique d'administration de la Bétahistine et de ses dérivés, son procédé de préparation et médicaments comprenant cette nouvelle forme.

NOM du déposant: CHERQUI Jean, Sadia
DJIANE Alain, Claude

La présente invention a pour objet une nouvelle forme pharmaceutique permettant le traitement des bourdonnements d'oreilles et des vertiges du type syndrome de Ménières.
La forme pharmaceutique selon l'invention renferme un sel de 2 (méthyl-amino-éthyl) pyridine microencapsulé.
Cette nouvelle forme pharmaceutique sert de médicament à action prolongée et à meilleure tolérance.

EP 0 016 890 A1

ACTORUM AG

COMPLETE DOCUMENT

0016890

2

La présente invention a pour objet une nouvelle forme phai...aceutique permettant le traitement des bourdonnements d'oreilles et des vertiges du type syndrome de Ménières.

On sait que ce type de symptômes atteint principalement les personnes âgées et que son traitement nécessite des cures prolongées requérant une parfaite tolérance du produit.

Le principal produit utilisé jusqu'ici pour le traitement de ces manifestations est le dichlorhydrate de 2 (méthyl-amino-éthyl) pyridine ou dichlorhydrate de Bétahistine. Toutefois ce produit, malgré sa grande valeur thérapeutique, n'a pu trouver une place correspondante sur le marché en raison des précautions d'emploi ou des actions indésirables que son utilisation entraîne. En outre, la durée d'action de ce produit est de courte durée et nécessite le recours à de nombreuses prises quotidiennes. En effet, la posologie quotidienne peut atteindre 48 mg/jour répartis en 6 prises.

On sait, en effet, que la bétahistine a une action de type histaminique sur les cellules sécrétrices de la muqueuse gastrique et que son emploi est déconseillé à des patients ayant déjà eu un ulcère gastroduodénal ou souffrant d'hyperchlorydie gastrique.

Il était donc intéressant de pouvoir disposer d'une nouvelle forme pharmaceutique assurant une diffusion régulière et prolongée du principe actif tout en supprimant les risques d'intolérance.

Les formes pharmaceutiques selon l'invention évitent les inconvénients précités et permettent au médecin de disposer d'un nouvel agent thérapeutique dont la sécurité d'emploi et la moindre posologie lui permettent d'envisager un traitement au long cours.

Les formes pharmaceutiques selon l'invention sont caractérisées en ce qu'elles renferment du diméthane sulfonate de Bétahistine microencapsulé à l'intérieur de petites sphères dont la membrane est semi-perméable. Cette forme pharmaceutique libère progressivement, en fonction de l'élévation du pH des liquides digestifs, la bétahistine.

La membrane semi-perméable des compositions selon l'invention est constituée de couches successives concentriques de polymères métacryliques dont l'épaisseur conditionne la vitesse de libération du principe actif. Le principe actif est incorporé à ces microgranules sous forme d'une dispersion dans un excipient inerte et disposé dans la masse métacrylique ou de solutions de ces polymères dans un solvant volatil.

A titre d'exemple, l'excipient inerte pourra être de l'amidon, du talc, du lactose, du saccharose, de la silice colloïdale, des celluloses, du stéarate de magnésium, du phosphate de magnésium ou de l'alumine.

Le polymère métacrylique est de préférence celui vendu sous la marque Eudragit $^R$.

Les formes pharmaceutiques selon l'invention sont présentées sous forme de gélules transparentes ou opaques renfermant un nombre déterminé de microgranules ou de poudres, sous forme de suspensions buvables aromatisées ou non.

On peut penser que les gélules s'ouvrent dans l'estomac au contact des sucs digestifs et libèrent un nombre important de ces microgranules, ce qui tranforme l'unité de prise en autant de sous unités autonomes. Cette multiplication entraîne une grande dispersion réalisant ainsi une absorption satisfaisante et une durée d'action prolongée.

4

0016890

Les gélules selon l'invention renferment en général de IO à I5 mg de principe actif par prise et de préférence I2 mg.

La posologie journalière est de 2 à 3 gélules, de préférence 3 gélules. On constate donc que, grâce aux compositions selon l'invention, il est possible d'assurer le traitement des vertiges du type de Ménières avec une posologie réduite de 50% tout en limitant à l'extrême l'apparition d'actions indésirables. La durée du traitement s'échelonne de 6 semaines à 6 mois selon les indications thérapeutiques.

Les compositions selon l'invention ont été essayées dans le traitement du vertige de Ménières et dans les troubles de l'insuffisance circulatoire cérébrale. Ces traitements s'adressaient plus particulièrement à des malades âgés (70 ans et plus) néanmoins des malades plus jeunes ont été traités dans des conditions identiques.

Exemple de réalisation d'une forme pharmaceutique selon l'invention-

On procède à la fabrication d'un grain de départ à base d'amidon, de saccharose et d'acide stéarique.Les grains tamisés sont placés dans une turbine adéquate dont la vitesse et le temps de rotation sont définis de façon à obtenir des grains parfaitement sphériques. Un autre tamisage a lieu et, ensuite, un passage à l'étuve.

Une certaine quantité de ces grains est mouillée avec de l'alcool éthylique, puis est enrobée en turbine à l'aide d'une solution de diméthane sulfonate de bétahistine. Talc et acide stéarique sont ajoutés dans le but de faciliter l'enrobage. Les grains sont ensuite passés dans une étuve ventilée.

Cette opération est renouvelée plusieurs fois.

Lorsqu'elle est terminée, une solution alcoolique de polymères métacryliques est pulvérisée de façon à parfaire l'enrobage. Les micrograins obtenus sont séchés pendant 2 à 4 jours dans une étuve ventilée.

5

On procède ensuite au titrage des microgranules actifs et on incorpore la quantité nécessaire de granules neutres, non enrobés pour arriver à un titre final du mélange de 12 mg de Bétahistine pour 240 mg de microgranules. On procède ensuite à la répartition en gélules, chaque gélule contenant 240 mg de microgranules.

Exemple de fabrication pour I00.000 gélules.

microgranules_actifs
Diméthane sulfonate de Bétahistine...... :  1,200 kg
Eudragit $^R$, acide stéarique, talc,
saccharose, amidon de maïs, aérosil .... : 20,800 kg

microgranules_neutres
Saccharose, amidon de maïs, acide stéa
-rique ............................. :  2,000 kg
                                        ─────────
                                         24,000 kg

Adjuvants de fabrication
Eau distillée, éthanol à 95°, acétone.

Détermination de la vitesse de libération du diméthane sulfonate de Bétahistine.

La libération du diméthane sulfonate de Bétahistine est conforme aux limites suivantes :

. Ière heure : libération inférieure à 40%
. 4ème heure : libération inférieure à 80%
. 8ème heure : libération supérieure à 80%

Ces contrôles sont effectués dans des conditions bien déterminées et à l'aide d'un appareillage approprié, qui reproduisent les conditions rencontrées dans l'organisme.

Ainsi la libération à la Ière heure est testée avec un suc gastrique artificiel à pH I,5 et au terme de cette première heure, le suc gastrique est remplacé et les micrograins sont soumis à un suc intestinal artificiel de pH 7,2.

L'appareillage utilisé permet de maintenir une agitation constante et une température comprise entre 36,5° et 37,5° C.

Chaque fabrication de microgranules peut être

6

ainsi testée et son enrobage modifié en conséquence.

<u>Compte-rendu des essais cliniques.</u>

A- <u>Troubles de l'insuffisance circulatoire cérébrale.</u>

Les essais cliniques ont été effectués sur 40 malades selon la méthodologie du double aveugle et par rapport à un placebo.

La durée du traitement a été de I2 semaines se répartissant en deux séquences de 6 semaines séparées par 2 semaines d'interruption. Ces 2 semaines étaient nécessaires pour permettre au malade de ne plus dépendre de la thérapeutique précédente.

L'ordre d'administration des traitements a été déterminé par randomisation dont le code, qui nous avait été remis par le fabricant sous pli cacheté, n'a été ouvert qu'en fin d'étude.

La distribution des médicaments et leur prise a été réalisée de façon régulière, selon le même protocole, tout au long de l'essai. La standardisation du nursing, auquel nous attachons une grande importance dans l'évolution des thérapeutiques destinées à améliorer la circulation cérébrale de sujets sénescents, a été observée avec une grande rigueur.

La réalisation des tests psychométriques destinés à évaluer l'échelle de CRICHTON a été conduite dans le cadre même du service, suivant une méthodologie particulièrement éprouvée.

- <u>Cotation des résultats</u> -

<u>Ière Evaluation</u>

Les résultats ont été évalués sur un double plan clinique et psychique. Cette évaluation a été facilitée par l'utilisation de l'échelle quantitative de Crichton. L'étude systématique des différents paramètres a permis de mettre en évidence après chaque traitement quels ont été les critères les plus améliorés.

Nous avons coté les résultats en : Bon, Moyen, Echec, en nous basant sur, d'une part l'état clinique du malade et, d'autre part le nombre d'items améliorés.

BON :

L'ensemble des paramètres est en amélioration.

MOYEN :

Une amélioration est enregistrée tant sur le plan subjectif qu'objectif. L'évolution des différents paramètres traduit cette amélioration.

ECHEC :

Il y a persistance de l'asthénie physique et psychique, ce qui est réflété par la stabilité des tests, négatifs au départ, et une stagnation de l'état général clinique.

2°) Contrôle de la tolérance -

a) Tolérance biologique -

Dans le cadre de la surveillance classique et en fonction des caractéristiques déjà connues de la bétahistine, nous avons effectué, avant et après chacun des traitements, les examens suivants :

. Urée

. Glycémie

. Cholestérol

. Transaminases, SGOT/SGPT

. Numération globulaire

. Formule sanguine

b) Tolérance clinique -

Nous avons attaché une importance toute particulière à la surveillance cardio-vasculaire, aussi nous avons pratiqué un bilan complet :

. T.A. avant et après traitement

. Rythme cardiaque, avant, pendant et après traitement

. E.C.G. avant et après traitement

La surveillance gastro-entérologique, transit intestinal, nausées, vomissements et la surveillance neurologique, ont été conduits par le personnel soignant, en complément de l'observation de chaque paramètre effectué dans le cadre
de l'étude de l'efficacité.

- RESULTATS -

La durée du traitement suivi a été de 6 semaines dans les 2 groupes. Les résultats obtenus sont les suivants:

Ier Groupe : Séquence Placebo - Produit Actif

. Placebo = 5 résultats moyens, soit 25 %

15 échecs , soit 75 %

. Produit actif = 16 Bons résultats, soit 80 %

3 Moyens , soit 15 %

I Echec , soit 5 %

2ème Groupe : Séquence Produit Actif -Placebo

. Produit actif = 12 Bons résultats, soit 60 %

4 Moyens , soit 20 %

4 Echecs , soit 20 %

. Placebo = 20 Echecs , soit 100%

Le score est donc très nettement en faveur du produit actif qui
réalise au total :

. 28 Bons résultats, soit 70 %

. 7 Résultats moyens, soit 17,5 %

. 5 Echecs, soit 12,5 %

ANALYSE DES RESULTATS

40 patients ont été traités dans le cadre de cet essai contrôlé,
conduit en double aveugle par rapport à un placebo et suivant la
technique du "crossing-over". Ces modalités bien respectées, ont
eu l'avantage de ne nécessiter qu'un nombre restreint de sujets.

- II patients sont de sexe masculin, soit 27,5%
du total.

- 29 patients sont de sexe féminin, soit 72,5%
du total.

Ceci correspond au recrutement habituel de bon nombre de services de Gériatrie.

Au terme de cette expérimentation, un certain nombre de remarques s'imposent :

- aucun malade n'a interrompu son traitement pendant l'essai.

- nous n'avons pas observé d'effets secondaires particuliers.

La tolérance du produit a été généralement bonne, hormis quelques cas d'intolérance sans gravité.

Les symptômes les plus améliorés par la composition selon l'invention sont :

Séquence : Placebo - Produit actif :

| | |
|---|---|
| - Anxiété - Peur | : 77 % |
| - Stabilité émotionnelle | : 73,7 % |
| - Vivacité d'esprit - Confusion | : 68,5 % |
| - Dépression réactionnelle | : 64,3 % |
| - Vertiges | : 6I,5 % |
| - Appétit | : 6I,5 % |
| - Céphalées | : 54,55 % |
| - Sommeil | : 53 % |
| - Bourdonnements d'oreilles | : 50 % |

Séquence : Principe actif - Placebo:

| | |
|---|---|
| - Anxiété - Peur | : 86,7 % |
| - Fatigue | : 83,4 % |
| - Stabilité émotionnelle | : 73,3 % |
| - Vivacité d'esprit - Confusion | : 69,2 % |
| - Dépression réactionnelle | : 62,5 % |
| - Céphalées | : 62,5 % |
| - Orientation | : 57 % |
| - Vertiges | : 50 % |

Ces résultats indiquent, qu'il n'existe aucun doute sur une activité de la bétahistine au niveau de la circulation cérébrale.

Les résultats sont particulièrement intéressants en ce qui concerne l'état psychique des malades.

L'amélioration très significative de certains items tels : vertiges, fatigue, céphalées, sommeil, bourdonnements d'oreilles nous confirme son efficacité clinique sur les signes de l'insuffisance circulatoire cérébrale.

10

c) <u>Tolérance biologique</u> -

La tolérance biologique a été excellente dans tous les cas :

- aucune modification de la formule sanguine
- aucune variation de l'urée ou de la glycémie
- aucune perturbation des tests hépatiques.

B) <u>Traitement de la maladie de Ménières</u> -

L'expérimentation a porté sur 25 malades d'âge moyen 42,5 ans répartis entre 10 hommes et 15 femmes. Le choix s'est porté uniquement sur des sujets présentant un syndrome de Ménières dont l'ancienneté variait de 6 mois à 20 ans.

La posologie et le schéma thérapeutique ont été établis, d'une part en tenant compte du niveau d'activité de la bétahistine et, d'autre part en fonction de la forme galénique propre aux compositions selon l'invention.

La bétahistine a été administrée à raison de 3 gélules par jour dosées à 12 mg de diméthane sulfonate de bétahistine.

La durée du traitement a été fixée à 3 mois au moins. Cette durée a pu, dans certains cas, être portée à 6 et même 9 mois.

Critères d'évaluation.

La crise de Ménières se manifeste par 3 symptômes précis :

- vertiges
- bourdonnements d'oreilles
- surdité

Le vertige est un symptôme pur, rotatoire, avec impression de déplacement des objets autour du malade.

Il peut durer plusieurs heures et s'accompagne de signes neurovégétatifs spectaculaires.

L'appréciation de l'efficacité clinique a été basée sur de tels critères objectifs. On a réalisé avant et après chaque traitement un examen labyrinthique, et un audiogramme, et on a procédé à l'appréciation des signes cliniques suivants : vertiges, nausées, otalgie, céphalées et acouphènes.

Analyse des résultats -

Indépendamment de l'évaluation clinique, il est apparu nécessaire de codifier les différents paramètres cliniques précédemment cités comme : très bon, bon, assez bon, moyen et échec.

Parallèlement, on a accordé une importance toute spéciale à la surveillance gastro-entérologique ainsi qu'à la tolérance cardiovasculaire et neurologique des compositions selon l'invention.

Conclusions -

Au terme de l'expérimentation, on a pu constater qu'aucun malade n'a interrompu son traitement, que l'on a observé aucun effet secondaire et que la tolérance des compositions a été très bonne dans l'ensemble.

L'essai réalisé sur 25 malades a donné les résultats suivants :

. T.B. et B résultat     =  16 cas, soit 64 %

. A.B. et résultat moyen =   6 cas, soit 24 %

. Echec                  =   3 cas, soit 12 %

Les résultats obtenus permettent de constater que les compositions selon l'invention manifestent une très nette action thérapeutique, qu'elles sont très actives sur le syndrome de Ménières et que l'administration prolongée ne requière aucune protection particulière.

Parmi les symptômes retenus comme critères objectifs d'appréciation il convient de noter que les vertiges ont été améliorés dans 88% des cas, les nausées dans 91% des cas et que les otalgies ont été très améliorées dans tous les cas.

En moyenne, le délai d'action du produit est de 10 jours, la durée d'action du produit persiste pendant toute la durée du traitement.

Les compositions pharmaceutiques selon l'invention ont été, aux dires des malades, beaucoup mieux tolérées

12

que la forme comprimés usuelle et non retard.

La tolérance biologique a été excellente dans tous les cas.

## REVENDICATIONS

1°) de nouvelles compositions pharmaceutiques caractérisées en ce qu'elles renferment du diméthane sulfonate de méthylamino éthyl 2- pyridine microencapsulé à l'intérieur de petites sphères dont la membrane est semi-perméable.

2°) des compositions thérapeutiques selon la revendication I° dans lesquelles les microsphères sont conditionnées en gélules ou présentées en suspension dans un gel buvable.

3°) des compositions pharmaceutiques selon les revendications I° et 2° dans lesquelles la teneur en principe actif s'échelonne entre IO et I5 mg par prise.

4°) médicaments, notamment pour le traitement du vertige de Ménières, caractérisés en ce qu'ils comprennent la nouvelle forme galénique d'administration du diméthane sulfonate de méthyl amino éthyl 2- pyridine selon la revendication I°.

0016890

# R E V E N D I C A T I O N S

1) Procédé d'obtention d'une nouvelle composition pharmaceutique renfermant du diméthanesulfonate de (méthylamino éthyl)-2 pyridine caractérisé en ce qu'on forme un granule neutre constitué par des excipients inertes et de l'acide stéarique sur lequel on dispose des strates successives et alternées de principe actif par pulvérisation d'une solution de diméthanesulfonate de (méthylamino éthyl)-2 pyridine et de matériau d'enrobage à concentration décroissante puis répartit les granules enrobés ainsi obtenus dans des gélules.

2) Les compositions pharmaceutiques à base de diméthanesulfonate de (méthylamino éthyl)-2 pyridine obtenues selon le procédé de la revendication 1.

Office européen
des brevets

**RAPPORT DE RECHERCHE EUROPEENNE**

0016890
Numéro de la demande

EP 79 40 0195

| Catégorie | DOCUMENTS CONSIDERES COMME PERTINENTS | | |
|---|---|---|---|
| | Citation du document avec indication, en cas de besoin, des parties pertinentes | Revendica-tion concernée | |
| X | FR - A - 2 397 840 (J.S. CHERQUI et al.)<br><br>* Page 1, ligne 1 - page 9, ligne 20; revendications *<br><br>---- | 1-4 | |

**CLASSEMENT DE LA DEMANDE (Int. Cl. 3)**

A 61 K 9/52
A 61 K 31/44

**DOMAINES TECHNIQUES RECHERCHES (Int. Cl. 3)**

A 61 K 9/52
A 61 K 31/44

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achevement de la recherche | Examinateur |
|---|---|---|
| La Haye | 23-11-1979 | WILLEKENS |

OEB Form 1503.1   06.78